# EUROPEAN PATENT SPECIFICATION

(11) **EP 2 743 282 B1**
(45) Date of publication and mention of the grant of the patent: **29.04.2020**
(21) Application number: 12196479.5
(22) Date of filing: 11.12.2012
(51) Int. Cl.: C08G 12/32, C07C 273/18, C07D 251/18, C08G 14/10

(54) **Melamine-aldehyde-condensation product and method obtaining the same**
Melamin-Aldehyd-Kondensierungsprodukt und Verfahren zu dessen Erhalt
Produit mélamine-aldéhyde-condensation et son procédé d'obtention

(43) Date of publication of application: 18.06.2014
(73) Proprietor: Borealis Agrolinz Melamine GmbH, 4021 Linz (AT)
(72) Inventor: Dicke, René, 4060 Leonding (AT); Gabriel, Herbert, 4076 Marienkirchen (AT)
(74) Representative: Maikowski & Ninnemann Patentanwälte Partnerschaft mbB

(56) References cited:
- EP-A1- 2 360 149
- WO-A1-2007/096200
- WO-A1-2009/121603
- DE-C- 905 850
- WICKS ZENO W JR ET AL: "MODEL COMPOUND FOR MELAMINE FORMALDEHYDE RESINS", JCT. JOURNAL OF COATINGS TECHNOLOGY, PHILADELPHIA, PA, US, vol. 55, no. 702, 1 July 1983 (1983-07-01) , pages 29-34, XP009083542, ISSN: 0361-8773

## Description

The invention relates to a melamine aldehyde condensation product according to the preamble of claim 1, a method for obtaining the same according to claim 9, the use of the melamine aldehyde condensation products according to claim 13 and wood based panels comprising said product according to claim 14.

### Description

Melamine is an important raw material in the production of resins for the manufacture of laminates for surface finishing, moulding compounds, paints and glues. Melamine-formaldehyde-resins as products of a condensation of melamine and formaldehyde are characterized by exceptional hardness, high gloss, flame retarded, as well as heat and solvent resistance.

Melamine formaldehyde resins are mainly used in the wood industry as adhesives in wood panels, as impregnation resin in laminates, as linking agents or as composite material. These conventional resins are highly linked and provide therefore stiff and hard duroplastic materials. An unmodified melamine formaldehyde resin forms a dense network during the condensation. The degree of crosslinking depends thereby on different factors such as the melamine / formaldehyde ratio, temperature and pH-value.

Since melamine-formaldehyde resins are stiff, brittle and have a reduced flexibility they often cause problems in the processabilty and postforming or dimensional stability properties of laminates and other melamine-formaldehyde resin containing products. Due to the low flexibility the laminate may crack for instance when bending the laminate over the edge. The smaller the radius the more difficult is the postforming process. As mentioned a high brittleness of the resin may causes problems in processing. For instance, cutting tools are of limited life time due to the exposure by the hard surface and also the edges of the laminate surface can shatter or splinter during processing.

Different methods are known in order to circumvent these problems. For instance, a common way to increase the flexibility of a thermoset material is on the one hand to add plasticisers with a reduced functionality compared to melamine. Another approach for increasing flexibility and to reduce the degree of linkage is reducing the ratio of formaldehyde to melamine. This method is however hampered by reduced stability and solubility problems of the aqueous resin.

Alternative methods were also tested in the past in order to improve the thermoplastic processability of triazine-aldehyde-resins such as melamine formaldehyde resins.

For instance, it has been described to conduct the condensation reaction with already modified melamine derivatives for changing the properties of melamine resins. For instance the use of N-alkylated melamine derivatives for the production of polymer products and their condensation products with formaldehyde is known.

DE 10 2006 027 760 A1 describes a melamine derivative formaldehyde condensation product with thermoplastic properties. Alkylated melamine such as N,N',N",N"-tetramethyl melamine and N,N',N"-trimethyl melamine are reacted with formaldehyde to condensation products with a high degree of linear structures. The melamine derivatives used comprise in particular two secondary amino groups and one tertiary amino group. Thus, the melamine derivatives do not comprise on the one hand primary amino groups and on the other hand comprise secondary amino groups still having one hydrogen atom. The presence of two different secondary amino groups promotes the formation of linear oligomers and polymers in the condensation reaction. These linear chain molecules can slide along each other at appropriate temperatures, providing a resin which is meltable already at relative low temperatures between 70 to 130°C and a melt viscosity between 0.1 and 100 Pas. It is also described to add unsubstituted melamine with any of the specifically substituted melamine derivatives providing condensation products having a deviating degree of linear structures.

EP 852 241 A1 discloses amino resin compositions obtained by reacting one or a mixture of the following triazine derivatives of the following structure 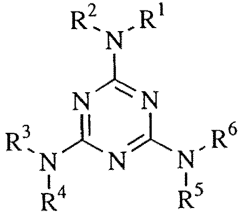 wherein R¹, R², R³, R⁴, R⁵ and R⁶ each represent an independent substituent and 1 to 5 substituents thereof represent for instance a C₁ to C₂₀ alkyl group or other moieties. The resins obtained are characterized by a good post forming ability and high hardness making them usable for laminated sheets, decorative sheets, moulding compounds and such alike.

It is also known to add other triazine derivatives such as guanamines like acetoguanamines or benzoguanamines to melamine formaldehyde resins for increasing the flowability of melamine formaldehyde resins or to improve the pre-hardening of the papers impregnated with such a resin mixture (see for example DE 44 39 156 A1) .

DE 102 51 653 A1 also discloses amino triazine copolymers with an improved water solubility wherein aldehydes are reacted with at least one derivative having the following structure 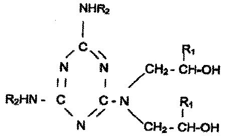 wherein R1 and R2 can be hydrogen. These types of resins are in particular suitable as an adhesive or an impregnating resin due to their high water solubility.

However, it is still a problem to provide a melamine aldehyde resin having a good flexibility and at the same time a sufficient high hardness and scratch resistance.

It was therefore an object of the present invention to provide a melamine aldehyde condensation product which combines scratch resistance with low brittleness and improved flexibility properties (e.g. improved postforming property, improved dimensional stability like reduced warpage and shrinkage, improved micro-scratch resistancy).

This object is being solved by providing melamine aldehyde condensation products comprising the features of claim 1.

Accordingly a melamine-aldehyde-condensation product is provided which is obtainable by reacting at least one aldehyde, in particular formaldehyde, with
a) Melamine,
b) at least one melamine derivative of the general structure (I) 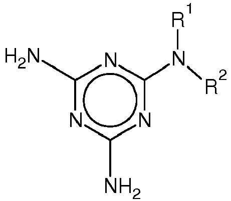 which is used in an amount of 1 to 45 mol% in respect to melamine,
   and
c) at least one melamine derivative of the general structure (II) 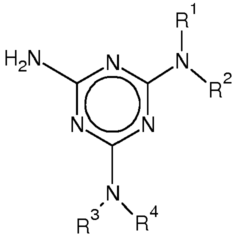
which is used in an amount of 1 to 45 mol% in respect to melamine,
wherein R¹, R², R³ and R⁴ mean independently from each other linear or branched C₁-C₂₀-alkyl, C₅-C₂₀-cyclo alkyl, C₅-C₂₀-aryl, C₁-C₂₀-alkylsubstituted C₅-C₂₀-aryl or cyclic amines, or wherein R¹ and R² or R³ and R⁴ form in each case together an alkyl ring, wherein in each case one or multiple carbon atoms can be substituted by one or multiple oxygen atoms, sulphur atoms and/or substituted nitrogen atoms and/or by one or multiple groups of the type -C(O)O-, -OC(O)-, -C(O)- and/or -OC(O)O-, and/or can be functionalized by one or multiple hydroxyl groups and/or mercapto groups; or an amide of a carboxylic acid or an imide of a cyclic dicarboxylic acid, and
wherein R¹, R², R³ and R⁴ can be the same or different,
wherein the molar ratio of aldehyde and melamine / melamine derivatives is between 1.8 : 1 and 1.3 : 1.

Thus, melamines with one or two blocked amino groups can be used as comonomers for a melamine-aldehyde resin, in particular melamine-formaldehyde resin, and/or modifier for lowering the network density of the resin and thereby increasing flexibility of the resin.

The melamine derivatives of formulae (I) comprise two primary amino group and one tertiary amino group and the melamine derivatives of formulae (II) comprise one primary amino group and two tertiary amino groups. Thus, no melamine derivative having a secondary amino group of the type -NHR is being used for obtaining the melamine-aldehyde condensation product.

This specific substitutional pattern of the melamine derivatives allows only a reaction of the aldehyde with the primary amino groups either of the melamine or the melamine derivatives. This also allows for adjusting the flexibility by choosing a suitable defined ratio of melamine and the melamine derivatives as well as the ratio of the melamine derivatives of formulae (I) and the melamine derivatives of formulae (II).

In an embodiment R¹, R², R³ and R⁴ are selected from a group comprising linear or branched C₁-C₁₀-alkyl, C₅-C₁₀-cycloalkyl, or C₁-C₁₀-alkylsubstituted C₅-C₁₀-aryl or wherein R¹ and R² or R³ and R⁴ form in each case together an C5-C10 alkyl ring, wherein in each case one or multiple carbon atoms can be substituted by one or multiple oxygen atoms, sulphur atoms and/or substituted nitrogen atoms and/or can be functionalized by one or multiple hydroxyl groups and/or mercapto groups, such as hydroxyethyl or mercaptoethyl. In the latter case the N-atom is therefore substituted by an ether type moiety. In case R¹ and R² or R³ and R⁴ form together an alkyl ring this ring can be a morpholine ring.

It is preferred, if in case the substituents representing the moities R¹, R², R³ and R⁴ are interrupted by one or multiple oxygen atoms, then the oxygen atom has at least a distance of (-CH2-)ₙ with n≥2, such as (-CH2-CH2-) from the N-atom, such as morpholine, ethylenglycol or diethylenglycol,

In a further embodiment the present melamine-aldehyde-condensation comprises moities R¹, R², R³ and R⁴ which are selected from a group comprising methyl, ethyl, n-propyl, isopropyl, n-butyl, s-butyl, t-butyl, isobutyl, pentyl, cyclopentyl, hexyl, cyclohexyl, 2-ethyl-hexyl, octyl, decyl, stearyl, phenyl, toloyl, xyloyl, hydroxyethyl, hydroxypropyl or wherein R¹ and R² or R³ and R⁴ are in each case part of morpholine.

It is in particular preferred if the melamine derivatives of formulae (I) and (II) are selected from a group comprising N,N-dialkylmelamine and N,N,N',N'-tetralkylmelamine, in particular N,N-dimethylmelamine, N,N,N',N'-tetramethylmelamine, N,N-diethylmelamine, N,N,N',N'-tetraethylmelamine, N,N-n-propylmelamine, N,N,N',N'-n-propylmelamine and others.

It is also particular preferred if the melamine derivatives of formulae (I) and (II) are selected from a group comprising N-morpholino melamine, N,N'-dimorpholino melamine, N,N-di(hydroxyethyl) melamine, N,N,N',N'-tetra(hydroxyethyl) melamine, N-hydroxyethyl-N-methyl melamine, N,N,N',N'-di-(hydroxyethyl-methyl) melamine, N-hydroxyethyl-N-ethyl melamine, N,N,N',N'-di-(hydroxyethyl-ethyl) melamine.

The melamine derivatives of formulae (I) and (II) can be obtained using different synthesis reactions. For instance they may be obtained by conversion of melamine with methyl amine salts (IG Farben, Nissan), catalytic hydrogenation of methyl etherified melamine formaldehyde resins using suitable catalysts such as Raney nickel (Casella), reductive alkylation of melamine by converting melamine with aldehydes / ketones in the presence of a suitable catalyst and hydrogen (Nissan), or alkylation of melamine with alkyl halogenids using a basic catalyst (Nissan 1995).

It is however mostly preferred if the melamine derivative of formulae (I) and (II) are obtained by a transamination reaction of the following type:
reacting melamine with at least one secondary amine of the general formulae (IIIa)

(R¹R²)NH

and/or the general formulae (IIIb)

(R³R⁴)NH

Wherein R¹, R², R³ and R⁴ have the above meanings, in the presence of at least one compound of the general formulae (IV)

(R⁵R⁶)₂NH₂⁺]ₙXⁿ⁻

wherein R⁵ and R⁶ are H, linear or branched C₁-C₂₀-alkyl, C₅-C₂₀-cycloalkyl, or C₁-C₂₀-alkylsubstituted C₅-C₂₀-aryl or cyclic amines or wherein R⁵ and R⁶ together form an alkyl ring, wherein in each case one or multiple carbon atoms can be substituted by one or multiple oxygen atoms, sulphur atoms and/or substituted nitrogen atoms and/or by one or multiple groups of the type -C(O)O-, -OC(O)-, -C(O)- and/or -OC(O)O- and/or can be functionalized by one or multiple hydroxyl groups and/or mercapto groups, wherein R⁵ and R⁶ can be the same or different,
wherein R¹, R², R³, R⁴, R⁵ and R⁶ can be the same or different, for the case that R⁵ and R⁶ are not H,
wherein Xⁿ⁻ is an anion of an organic or inorganic acid, and
wherein n ≥1, in particular 1, 2, 3 or 4.

It is further preferred if the at least one melamine derivative of formulae (I) is used in an amount of 2 to 25 mol%, in particular preferably 3 to 10 mol% in respect to melamine. Thus, the amount of the melamine derivative of formulae (I) used is less than the amount of melamine used. This specific ratio between melamine and melamine derivative of formulae (I) provides a consensus for a resin with an optimum between flexibility of the resin and postforming properties, hardness or scratch resistancy of the final products, like laminates. The main reason for this tailor-made property is the relationship to the resin structure regarding the available reactive aminogroups per triazine molecules which provides the right ratio of reactive groups to aldehyde.

It is also preferred if the at least one melamine derivative of formulae (II) is used in an amount of 2 to 25 mol%, in particular preferably 2 to 10 mol% in respect to melamine. Thus, the amount of the melamine derivative of formulae (II) used is less than the amount of melamine, but can be the same, less or even higher than the amount of the melamine derivative of formulae (I).

Furthermore, if in case melamine derivatives of formulae (I) and (II) are combined and are reacted together with the aldehyde then the ratio between the melamine derivative of formulae (I) and (II) may be in a range between 9:1 and 1:3, preferably between 4:1 and 1:1, most preferably in a range between 4:1 and 2:1. Thus, the melamine derivative of formulae (I) may be used in excess to the melamine derivative of formulae (II), but also in equal amounts or lower amounts than the melamine derivative of formulae (II).

It is in particular preferred if the melamine derivative of formulae (I) and (II) are used in form of a product mixture as obtained after their synthesis by transamination as described above. This has the advantage that it is possible to use directly a mixture of melamine derivatives of formulae (I) and (II) the ratio thereof being already adjustable during transamination synthesis by choosing the appropriate transamination conditions. For instance, the ratio of both melamine derivatives may be adjusted during their synthesis by choosing a suitable ratio of reactants, temperature, amount of catalyst or reaction time.

As describe above the molar ratio of aldehyde and melamine / melamine derivatives used in the synthesis is between 1.8:1 and 1.3:1. As an example, the aldehyde to melamine/melamine derivative ratio may be 1.59:1.

The present melamine aldehyde condensation products used in moulding mass of typ MF 152 according to DIN EN ISO 14528 may have a bending elongation between 2.0 and 3.0 %, preferably between 2.2 and 2.8 %, most preferably between 2.2. and 2.5 %. The present melamine aldehyde condensation product may have impact strength between 8.0 and 9.0 kJ/m², preferably between 8.2 and 9.0 kJ/m². The notch impact strength is between 1.8 and 2.0 kJ/m². The bending elongation and impact strength can be influenced by the amount of melamine derivatives of formulae (I) and/or (II) are added to the product. For instance, the higher the amount of melamine derivative added the higher the bending elongation.

The moulding shrinkage of the present melamine aldehyde condensation products is between 0.3 and 0.9 %, preferably between 0.3 and 0.7 % and the post shrinkage between 0.5 and 1.2 %, preferably between 0.5 and 1.1 %

The formability radius of the present melamine aldehyde condensation products as determined by the hot-mandrel test is between 1 an 4 mm, preferably between 1 and 3 mm, most preferably 2 and 3 mm. The formability of the present melamine aldehyde condensation products can thereby be influenced by the amount and type of melamine derivative added. For instance, the higher the amount of melamine derivative, the smaller the formability radius. This in turns mean that the melamine aldehyde condensation products with a high amount of melamine derivatives can be easily formed and the more flexible the condensation product is.

The reaction is now exemplarily shown using melamine, N,N-dimethylmelamine (N,N-DMM) and N,N,N',N'-tetramethylmelamine (N,N,N',N'-TMM).

Here a mixture of N,N-DMM and N,N,N',N'-TMM obtained from the transamination of melamine with dimethylamine have blocked amino-groups (shown as ellipses) and are used as comonomer for melamine-formaldehyde-resin synthesis. The resin formation occurs using the remaining non-blocked amino-groups (shown as circles). The flexibility is adjustable by the relation between melamine and methylated melamines. The mixture of methylated melamine derivatives (DMM+TMM) are preferably added in a range from 1 % to 25 % to the MF-resin. The preferred ratio in percentage between DMM and TMM is between 90:10 and 10:90, most preferred between 80:20 and 50:50. In the particular case shown below the ratio of N,N-DMM and N,N,N',N'-TMM is 1:1.

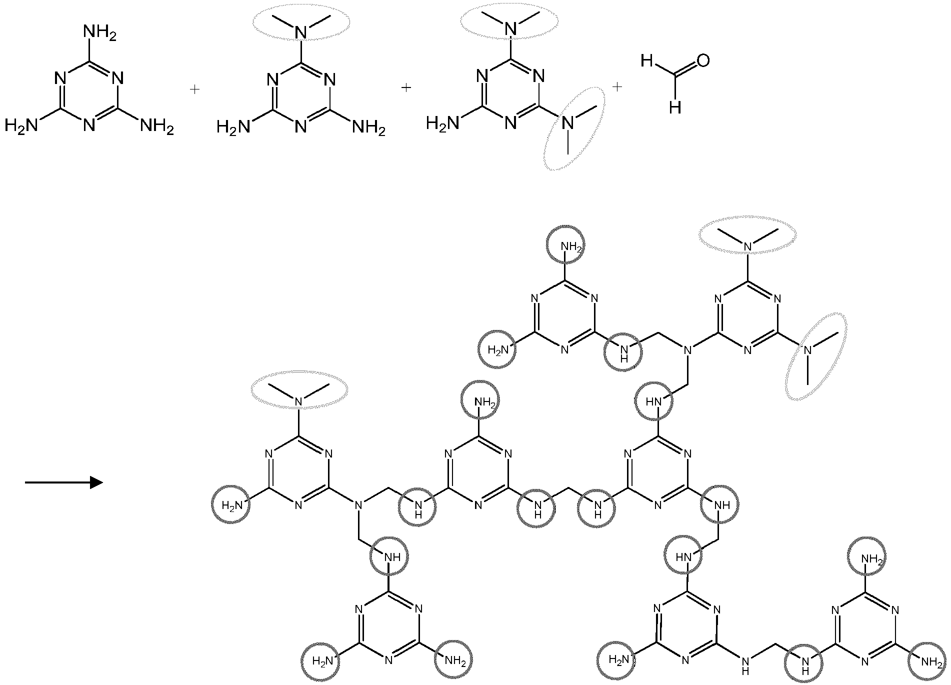

As clearly deducible from the above reaction scheme the specific substitutional pattern of the melamine derivatives used, i.e. no secondary amino groups, in conjunction with the aldehyde/melamine -melamine derivative ratio provides a resin with a rather low degree of linkage. This is due to the fact that when in particular using a aldehyde/melamine ratio of 1.3:1 to 2:1 the aldehyde reacts preferably only once per amino group. If now one or two of the amino groups are blocked then the aldehyde is not able to react with any of these blocked amino groups and the condensation is simply interrupted. The blocked amino groups also function as a type of distance holder due to their steric hindrance or shielding. Thus, the oligomers can not react as easily with one another during polycondensation and cannot form large networks. A further precondensation or polycondensation is only possible via the originally primary amino group and/or the secondary amino groups of the methylene bridges formed from the primary amino groups (shown as circles).

It is to be understood that above reaction is only one possible example and that the present invention is not restricted to these specific compounds but that rather multiple different melamine derivatives may be used as described in detail above.

The synthesis of the present melamine-aldehyde-condensation products using the melamine derivatives of formulae (I) and (II) is in general surprising and was not expected to be possible since the melamine derivatives of formulae (I) and (II) are known to have a very low water solubility, in particular in contrast to those melamine derivatives comprising a secondary amino group or only primary amino groups as melamine itself. For example the water solubility of N,N-dimethylmelamine is 1.2 g/l in water at 20 °C, whereas the water solubility of N,N'-dimethylmelamine is 41.1 g/mol in water at 20 °C. The same is true for other melamine derivatives. For instance, N,N,N',N'-tetramethylmelamine has a water solubility of 0.4 g/l in water at 20 °C, whereas N,N,N',N"-tetramethylmelamine has water solubility of 49 g/l in water at 20 °C. Additionally, also the solubility of melamine with 3.2 g/l in water at 20 °C is slightly higher than the solubility of melamine derivatives of formulae (I) or (II).

The present melamine-aldehyde-condensation product, in particular melamine-aldehyde-precondensation product, may be obtained in a method comprising the steps of
- providing a mixture of at least one aldehyde and melamine
- adding simultaneously or at a later stage at least one melamine derivative of formulae (I) and/or at least one melamine derivative of formulae (II), preferably as a solid or as precondensate,
- adjusting the pH value of the mixture to a range between 7 and 11, preferably 8 and 10,
- heating the reaction mixture to a temperature between 60 °C and 120 °C, preferably 70 °C and 110 °C, most preferably 80 °C and 100 °C until a clear solution is obtained for example for at least 30min,
- stirring the resin mixture at elevated temperature until a water tolerance of the resin at 20 °C of v(water):v(resin) between 3:1 to 0.3:1, preferably 2.5:1 to 0.6:1, most preferably 2:1 to 1:1 is obtained, and
- cooling the reaction mixture to room temperature after completion of the reaction, and
- optionally and if applicable, adjusting finally the pH value to a value range of 8 to 10.

As mentioned the melamine derivative of formulae (I) and (II) may be added and mixed simultaneously with the aldehyde and melamine. It is however also possible to add the melamine derivatives at a later stage to the aldehyde-melamine-mixture for instance after adjustment of the pH value or even heating the mixture.

It is preferred if the solvent is selected from a group comprising water, an alcohol or a mixture thereof, such as a mixture comprising 1-95 wt% water and 99-5 wt% alcohol like methanol, ethanol, isopropanol or such.

It is furthermore preferred if the aldehyde is selected from a group comprising a C₁-C₈ aldehyde such as formaldehyde, acetaldehyde, glyoxal, dimethoxyethanal.

It is furthermore preferred if auxiliary substances are added to the condensate. The auxiliary substances can be selected from a group comprising polyols, like ethylenglycol, diethyleneglycol, polyethyleneglycol, trimethylolpropane, sorbitol, dicyandiamide, urea, triethanolamine, caprolactam, sugar and/or o/p-toluene sulphonamide and others. The auxiliary substance may be added simultaneously with the melamine derivatives to the melamine-aldehyde-mixture or at a later stage.

In a variant of the present method the pH value is adjusted using an organic or inorganic base, preferably an inorganic base selected from a groups comprising NaOH, Na₂CO₃, KOH, K₂CO₃, NH₃ or alike.

The present melamine-aldehyde-condensation product can be used as a cross linker of a coating formulation or a coating such as furniture coating or as impregnation resin for laminate papers or for decor layers of postforming laminates, laminate flooring or 3D laminates, and adhesives such as adhesives for wood based panels. These laminates can be processed as a high pressure laminate (HPL), low pressure laminate (LPL) or a continuous pressure laminate (CPL).

Thus, melamine-aldehyde-condensation products of the present invention may be used as glues or binders in wood based panels, such as chipboards or particle boards (PB), OSB, MDF, LDF or HDF and plywood.

The use of the rather flexible melamine-aldehyde-condensation products in wood based panels as coating and/or adhesives allows for an overall improvement and increase of flexibility of the wood based panels obtained thereby. This in turn provides the possibility to produce wood based panels with a rounded or curved surface extending the use of such panels in the furniture industry.

Further details of the invention will be explained by the means of the following examples.

### Example 1

40.4 g melamine (1 mol equivalent), 43.4 g 30% formalin aqueous solution (1.59 mol equivalent in respect to melamine/melamine derivative) and 2.47 g N,N-dimethylmelamine (5 mol% in respect to melamine) and water are mixed at a temperature of 25 °C. The pH is adjusted to a value of 9.0±0.1 using 0.1 N NaOH. The mixture is heated to 93 °C at a speed of 5 K/min followed by heating to a temperature of 98 °C, is hold at this temperature until the clearing point (e.g. the point at which all melamine and melamine derivative are dissolved) is reached (38 minutes in this case) and is subsequently cooled again to 93 °C with a rate of 2.5 K/min. The reaction mixture is cooled to 20 °C after reaching a water tolerance of 2.0. The reaction product has a storage stability of 36 days at 25 to 28 °C.

### Example 2

40.4 g melamine (1 mol equivalent), 43.4 g 30% formalin aqueous solution (1.59 mol equivalent in respect to melamine/melamine derivatives), 2.0 g N,N-dimethylmelamine (4 mol% in respect to melamine), 0.6 g N,N,N',N'-tetramethylmelamine (1 mol% in respect to melamine) were reacted under the same conditions as described in example 1. The resin obtained has a storage stability of about 16 days at 25 to 28 °C.

### Example 3

40.4 g melamine (1 mol equivalent), 43.4 g 30% formalin aqueous solution (1.59 mol equivalent in respect to melamine/melamine derivatives), 1.75 g N,N-dimethylmelamine (3.5 mol% in respect to melamine), 0.9 g N,N,N',N'-tetramethylmelamine (1.5 mol% in respect to melamine) and water are mixed and reacted under the same reaction condition as described in example 1. The resin obtained has a storage stability of about 10 days at 25 to 28 °C.

### Example 4

40.4 g melamine (1 mol equivalent), 46.8 g 30% formalin aqueous solution (1.7 mol equivalent in respect to melamine/melamine derivatives), 1.5 g N,N-dimethylmelamine (3 mol% in respect to melamine), 1.2 g N,N,N',N'-tetramethylmelamine (2 mol% in respect to melamine) and water are mixed and reacted under the same reaction conditions as described in example 1. The resin obtained has a storage stability of 5 days at 25 to 28 °C.

### Example 5

38.4 g melamine (0.95 mol equivalent), 43.4 g 30% formalin aqueous solution (1.59 mol equivalent in respect to melamine/melamine derivative), 4.94 g N,N-dimethylmelamine (10 mol% in respect to melamine) and water are mixed and reacted under the same reaction conditions as described in example 1. The resin obtained has a storage stability of 25 days at 25 to 28 °C.

### Example 6

34.4 g melamine (0.85 mol equivalent), 43.4 g 30% formalin aqueous solution (1.59 mol equivalent in respect to melamine/melamine derivative), 9.88 g N,N-dimethylmelamine (20 mol% in respect to melamine) and water are mixed and reacted under the same reaction condition as described in example 1. The resin obtained has a storage stability of 15 days at 25 to 28 °C.

### Example 7

32.3 g melamine (0.8 mol equivalent), 43.4 g 30% formalin aqueous solution (1.59 mol equivalent in respect to melamine/melamine derivative), 12.35 g N,N-dimethylmelamine (25 mol% in respect to melamine) and water are mixed and reacted under the same reaction condition as described in example 1. The resin obtained has a storage stability of 12 days at 25 to 28 °C.

### Comparitive Example 8

1270 wt parts formalin (30%), 400 wt parts water and 35 wt parts diethyleneglycol are mixed and adjusted to a pH of 8.9 (20°C) using NaOH. After adding 1000 wt parts melamine and 35 wt parts acetoguanamine the mixture is heated to 90 °C in about 30 min. During the subsequent condensation at 90 °C the hydrophobic point (first cloudy turbidity when diluting with water) is reached after about 60 min. After further 40 min condensation at 90 °C a water tolerance of 1.7 is obtained. After reaching this condensation degree the resin solution is cooled rapidly.

### Comparative Example 9

40.4 g melamine (1 mol equivalent), 43.4 g formalin (1.67 mol equivalent in respect to melamine) and 4.74 g diethyleneglycol and water are mixed at a temperature of 25 °C. The pH is adjusted to a value of 9.0 ± 0.1 using 0.1 N NaOH. The mixture is heated to 93 °C at a speed of 5 K/min followed by heating to a temperature of 98 °C, is hold at this temperature until the clearing point (e.g. the point at which all melamine and melamine derivative are dissolved) is reached (28 minutes in this case) and is subsequently cooled again to 93 °C with a rate of 2.5 K/min. The reaction mixture is cooled to 20 °C after reaching a water tolerance of 2.0. The reaction product has a storage stability of 10 days at 25 to 28 °C.

### Comparative Example 10

40.4 g melamine (1 mol equivalent), 43.4 g 30% formalin aqueous solution (1.59 mol equivalent in respect to aminotriazine derivatives) and 2 g acetoguanamine (5mol% in respect to melamine) and water are mixed at a temperature of 25 °C. The pH is adjusted to a value of 9.0 ± 0.1 using 0.1 N NaOH. The mixture is heated to 93 °C at a speed of 5 K/min followed by heating to a temperature of 98 °C, is hold at this temperature until the clearing point (e.g. the point at which all melamine and melamine derivative are dissolved) is reached (36 minutes in this case) and is subsequently cooled again to 93 °C with a rate of 2.5 K/min. The reaction mixture is cooled to 20 °C after reaching a water tolerance of 2.0. The reaction product has a storage stability of 21 days at 25 to 28 °C.

### Example 11

### 11.1 Wet paper impregnation and drying of the impregnated papers

The fresh prepared resins are used for the production of the laminates. Per 400 g of resin solution of each Examples 1 to 10 (55% solid content) 0.5 w% wetting agent DeuroWet MA11 equivalent to 2 g and 0.5 w% parting agent DeuroLease PHE equivalent to 2 g are added. The viscosity and the clouding time of the solution were measured. The clouding time has to be in a range of 90-110 sec with a viscosity of approximately 100 mPas.

A white decor paper with a weight of 82 g/m² is used for wet paper impregnation. The paper was cut to a dimension of 32 x 28 cm per square.

The impregnation was performed with an impregnation machine of the company Munksjö decor under the following conditions. A simple impregnation with smooth blades was done. The impregnated papers were dried in a Mathis lab dryer at 120 °C for 1.5 minutes. The fan speed was set at 1600 rpm while to come to a required moisture content of approximately 6-7%. The resin content on the impregnated paper was determined. For all the papers a resin content of approximately 110 % was achieved.

### 11.2 Production of the laminates

The laminates were obtained by one layer impregnated decor paper and on two layers of phenol resin impregnated kraft papers (Hans Schmid KG; 155/228-233 postforming kraft paper). The papers have been pressed at 160 °C at a pressure of 67 bars for 30 seconds.

### 11.3 Formability (hot-mandrel) test according to ISO 4586/2

The test specimen (200 mm x 50 mm) was bent around the hot mandrel (170 °C) to an angle of 120° within 60 seconds. Different mandrels with radii between 10 and 2 mm were used. The sample was removed after forming and cooled, dried and inspected for signs of failure. The evaluation of the performance is described by the radius which is the first with visible signs of failure.

### 11.4 Acid Test according to DIN EN 438 (part II)

For this purpose a Petri dish with 0.2 N HCI, stained with 10 mg of Rhodamine B, is applied for 24 h on the pressed laminate. After 24 h, the 0.2 N HCI is washed and the laminate is dried. The performance is described by a school grade of 1 to 5

### 11.5 Reaction to water vapour according to DIN EN 438 (part II)

A specimen with a dimension of 10 x 10 cm per square is placed over the neck of a bottle with boiling water so that the decorative surface is exposed to water vapour. After 1 h the specimen is removed and stored for 24 h at room temperature before being examined for changes in appearance. The performance is described by a school grade of 1 to 5.

**Table 1**

| Example | formability radius in mm | acid test | water vapour test |
|---|---|---|---|
| 1 | 4 | 1 | 1 |
| 2 | 3 | 1 | 1 |
| 3 | 3 | 1 | 1 |
| 4 | 2 | 2 | 1 |
| 5 | 2 | 2 | 1 |
| 6 | 2 | 2 | 2 |
| 7 | 2 | 3 | 2 |
| 8 | 5 | 1 | 1 |
| 9 | 10 | 1 | 1 |
| 10 | 4 | 1 | 1 |

As clearly deducible from the data listed in Table 1 the formability (2-4 mm radius) of a laminate impregnated with the present melamine-aldehyde-condensation product is improved, in particular when using a relative high amount of melamine derivative (Examples 2-7).

### Example 12

### 12.1 Preparation of moulding masses

After freeze drying of the aqueous resin synthesis product of each Example of 1 to 10 the moulding compounds were prepared by using a lab kneader of ThermoElectron with the kneading chamber 3000p at 120 °C using Banbury rotor at a speed of 70 rpm. The moulding is pre-mixed at a lab blender based on a recipe of 51 mass-% of melamine resin of Example 1 to 10, 30 mass-% of cellulose fibre Arbocel FD-00, 15 mass-% Calcium carbonate and 4 mass-% of a mixture of lubricant, mould release agent and curing agent. This mixture is added into the kneading chamber during a time frame of 2-3 min and subsequently kneaded for 4-5 min. The kneaded moulding mass is taken out, cooled down and milled in an impact mill SM 2000 of company Retsch.

### 12.2 Preparation of test specimen by injection moulding

The test specimens of these moulding masses are prepared in an injection moulding machine of Thermo Electron, Minijet. The powder is molden in the injection unit at 70 ^ °C for 1 min. At next the molden material is injected into a 180 °C hot tool (specimen dimension 80x10x2mm). After 60 s the tool was opened and the test specimen is removed. These test specimens are now tested, for each test 10 specimens were used and the average value calculated.

**Table 2**

| Example | Flexural modulus GPa | Bending strength MPa | Bending elongation % | Impact strength kJ/m² | Notch impact strength kJ/m² | Moulding shrinkage % | Post shrinkage % |
|---|---|---|---|---|---|---|---|
| | ISO 178 | | | ISO 179 | | ISO 2577 | |
| 1 | 6.4 | 126 | 2.2 | 8.2 | 1.8 | 0.9 | 1.2 |
| 2 | 6.3 | 125 | 2.2 | 8.2 | 1.8 | 0.8 | 1.2 |
| 3 | 6.3 | 126 | 2.2 | 8.4 | 1.8 | 0.7 | 1.1 |
| 4 | 6.2 | 124 | 2.3 | 8.6 | 1.9 | 0.7 | 0.9 |
| 5 | 6.0 | 120 | 2.4 | 8.8 | 1.9 | 0.7 | 1.0 |
| 6 | 5.8 | 116 | 2.4 | 8.7 | 1.9 | 0.6 | 0.8 |
| 7 | 5.6 | 112 | 2.5 | 9.0 | 2.0 | 0.5 | 0.8 |
| 8 | 6.6 | 121 | 1.9 | 7.5 | 1.6 | 1.2 | 1.5 |
| 9 | 7.1 | 122 | 1.7 | 7.0 | 1.5 | 1.3 | 1.6 |
| 10 | 6.5 | 118 | 1.8 | 7.3 | 1.5 | 1.0 | 1.3 |

As clearly can be seen in Table 2 the elongation and overall strength of the present melamine-aldehyde product (Example 1-7) is increased in comparison to the comparative examples (Examples 8-10) having no melamine derivatives incorporated into the resin.

## Claims

1. Melamine-aldehyde-condensation product obtainable by reacting at least one aldehyde, in particular formaldehyde, with
a) Melamine,
b) at least one melamine derivative of the general structure (I) 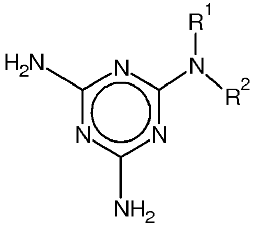 which is used in an amount of 1 to 45 mol% in respect to melamine,
and
c) at least one melamine derivative of the general structure (II) 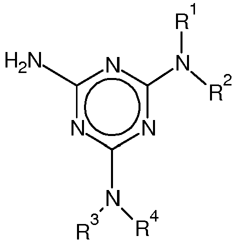
which is used in an amount of 1 to 45 mol% in respect to melamine,
wherein R¹, R², R³ and R⁴ mean independently from each other linear or branched C₁-C₂₀-alkyl, C₅-C₂₀-cyclo alkyl, C₅-C₂₀-aryl, C₁-C₂₀-alkylsubstituted C₅-C₂₀-aryl or cyclic amines, or wherein R¹ and R² or R³ and R⁴ form together an alkyl ring, wherein in each case one or multiple carbon atoms can be substituted by one or multiple oxygen atoms, sulphur atoms and/or substituted nitrogen atoms and/or by one or multiple groups of the type -C(O)O-, -OC(O)-, -C(O)- and/or -OC(O)O-, and/or can be functionalized by one or multiple hydroxyl groups and/or mercapto groups; or an amide of a carboxylic acid or an imide of a cyclic dicarboxylic acid, and
wherein R¹, R², R³ and R⁴ can be the same or different,
wherein the molar ratio of aldehyde and melamine / melamine derivatives is between 1.8 : 1 and 1.3 : 1.

2. Melamine-aldehyde-condensation product according to claim 1, **characterized in that** R¹, R², R³ and R⁴ are selected from a group comprising linear or branched C₁-C₁₀-alkyl, C₅-C₁₀-cycloalkyl, or C₁-C₁₀-alkylsubstituted C₅-C₁₀-aryl or wherein R¹ and R² or R³ and R⁴ form in each case together an C₅-C₁₀ alkyl ring, wherein in each case one or multiple carbon atoms can be substituted by one or multiple oxygen atoms, sulphur atoms and/or substituted nitrogen atoms and/or can be functionalized by one or multiple hydroxyl groups and/or mercapto groups.

3. Melamine-aldehyde-condensation product according to claim 1 or 2, **characterized in that** R¹, R², R³ and R⁴ are selected from a group comprising methyl, ethyl, n-propyl, isopropyl, n-butyl, s-butyl, t-butyl, isobutyl, pentyl, cyclopentyl, hexyl, cyclohexyl, 2-ethyl-hexyl, octyl, decyl, stearyl, phenyl, toloyl, xyloyl, hydroxyethyl, hydroxypropyl or wherein R¹ and R² or R³ and R⁴ are part of morpholine.

4. Melamine-aldehyde-condensation product according to one of the preceding claims, **characterized in that** the at least one melamine derivative of formulae (I) is used in an amount of 2 to 25 mol%, in particular preferably 2 to 10 mol% in respect to melamine.

5. Melamine-aldehyde-condensation product according to one of the preceding claims, **characterized in that** the at least one melamine derivative of formulae (II) is used in an amount of 2 to 25 mol%, in particular preferably 2 to 10 mol% in respect to melamine.

6. Melamine-aldehyde-condensation product according to one of the preceding claims, **characterized in that** if in case a melamine derivative of formulae (I) and (II) are used together then the ratio between the melamine derivative of formulae (I) and (II) is in a range between 9:1 and 1:3, preferably between 4:1 and 1:1, most preferably in a range between 4:1 and 2:1.

7. Melamine-aldehyde-condensation product according to claim 6, **characterized in that** the melamine derivative of formulae (I) and (II) are used in form of a product mixture as obtained after their synthesis, in particular synthesis by transamination.

8. Melamine-aldehyde-condensation product according to one of the preceding claims, **characterized by** a formability radius as determined by the hot-mandrel test between 1 an 4 mm, preferably between 1 and 3 mm, most preferably 2 and 3 mm.

9. Method for obtaining a melamine-aldehyde-condensation product according to one of the preceding claims comprising the steps of
- providing a mixture of at least one aldehyde and melamine,
- adding simultaneously or at a later stage at least one melamine derivative of formulae (I) and/or at least one melamine derivative of formulae (II),
- adjusting the pH value of the mixture to a range between 7 and 11, preferably 8 and 10,
- heating the reaction mixture to a temperature between 60 °C and 120°C, preferably 70°C and 110°C, most preferably 80 °C and 100°C until a clear solution is obtained,
- stirring the resin mixture at elevated temperature until a water tolerance of the resin at 20°C of v(water):v(resin) between 3:1 to 0.3:1, preferably 2.5:1 to 0.6:1, most preferably 2:1 to 1:1 is obtained, and
- cooling the reaction mixture to room temperature after completion of the reaction, and
- optionally adjusting the pH value to a range between 8 and 10.

10. Method according to claim 9, **characterized in that**, the solvent is selected from a group comprising water, an alcohol or a mixture thereof, such as a mixture comprising 1-95wt% water and 99-5 wt% alcohol, in particular methanol, ethanol, isopropanol.

11. Method according to claim 9 or 10, **characterized in that** the aldehyde is selected from a group comprising a C1-C8 aldehyde, in particular formaldehyde, acetaldehyde, glyoxal. dimethoxyethanal.

12. Method according to one of the claims 9 to 11, **characterized in that** the pH value is adjusted using an organic or inorganic base, preferably an inorganic base selected from a groups comprising NaOH, KOH, Na₂CO₃, K2CO₃, NH3.

13. Use of a melamine-aldehyde-condensation product according to claim 1 to 8 as an impregnation resin for laminate papers and as coating such as laminate coating or for decor layers of postforming laminates, adhesives such as adhesives for wood based panels.

14. Wood based panels, in particular particle boards (PB), OSB, MDF, LDF or HDF and plywood, comprising a melamine-aldehyde-condensation product according to one of the claims 1 to 8.

## Patentansprüche

1. Melamin-Aldehyd-Kondensationsprodukt erhältlich durch Reaktion von mindestens einem Aldehyd, insbesondere Formaldehyd, mit
a) Melamin,
b) mindestens ein Melaminderivat der allgemeinen Struktur (I), 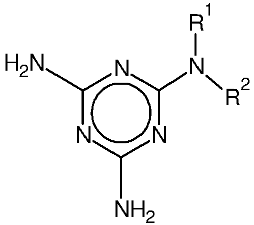 das in einer Menge von 1 bis 45 Mol% in Bezug auf Melamin verwendet wird, und
c) mindestens ein Melaminderivat der allgemeinen Struktur (II), 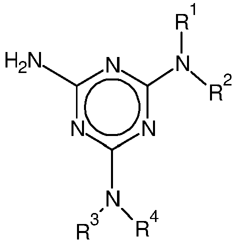
das in einer Menge von 1 bis 45 Mol% in Bezug auf Melamin verwendet wird,
worin R¹, R², R³ und R⁴ unabhängig voneinander lineares oder verzweigtes C₁-C₂₀-Alkyl, C₅-C₂₀-Cycloalkyl, C₅-C₂₀-Aryl, C₁-C₂₀-alkylsubstituiertes C₅-C₂₀-Aryl oder cyclische Amine bedeuten, oder worin R¹ und R² oder R³ und R⁴ zusammen einen Alkylring bilden, wobei jeweils ein oder mehrere Kohlenstoffatome durch ein oder mehrere Sauerstoffatome, Schwefelatome und/oder substituierte Stickstoffatome und/oder durch eine oder mehrere Gruppen vom Typ -C(O)O-, -OC(O)-, -C(O)- und/oder -OC(O)O- substituiert sein können und/oder durch eine oder mehrere Hydroxylgruppen und/oder Mercaptogruppen funktionalisiert sein können; oder ein Amid einer Carbonsäure oder ein Imid einer zyklischen Dicarbonsäure, und
wobei R¹, R², R³ und R⁴ gleich oder verschieden sein können,
wobei das Molverhältnis von Aldehyd und Melamin/Melaminderivaten zwischen 1,8 : 1 und 1,3 liegt: 1.

2. Melamin-Aldehyd-Kondensationsprodukt nach Anspruch 1, **dadurch gekennzeichnet, dass** R¹, R², R³ und R⁴ ausgewählt sind aus einer Gruppe, die lineares oder verzweigtes C₁₋C₁₀-Alkyl, C₅-C₁₀-Cycloalkyl oder C₁C₁₀-alkylsubstituiertes C₅-C₁₀-Aryl umfasst oder worin R¹ und R² oder R³ und R⁴ jeweils gemeinsam einen C₅-C₁₀)-Alkylring bilden, wobei jeweils ein oder mehrere Kohlenstoffatome durch ein oder mehrere Sauerstoffatome, Schwefelatome und/oder substituierte Stickstoffatome substituiert und/oder durch eine oder mehrere Hydroxylgruppen und/oder Mercaptogruppen funktionalisiert sein können.

3. Melamin-Aldehyd-Kondensationsprodukt nach Anspruch 1 oder 2, **dadurch gekennzeichnet, dass** R¹, R², R³ und R⁴ aus einer Gruppe ausgewählt sind, die Methyl, Ethyl, n-Propyl, Isopropyl, n-Butyl, s-Butyl, t-Butyl umfasst, Isobutyl, Pentyl, Cyclopentyl, Hexyl, Cyclohexyl, 2-Ethyl-Hexyl, Octyl, Decyl, Stearyl, Phenyl, Toloyl, Xyloyl, Hydroxyethyl, Hydroxypropyl oder worin R¹ und R² oder R³ und R⁴ Teil des Morpholins sind.

4. Melamin-Aldehyd-Kondensationsprodukt nach einem der vorstehenden Ansprüche, **dadurch gekennzeichnet, daß** das mindestens eine Melaminderivat der Formeln (I) in einer Menge von 2 bis 25 Mol-%, insbesondere vorzugsweise 2 bis 10 Mol-%, bezogen auf Melamin, verwendet wird.

5. Melamin-Aldehyd-Kondensationsprodukt nach einem der vorstehenden Ansprüche, **dadurch gekennzeichnet, daß** das mindestens eine Melaminderivat der Formeln (II) in einer Menge von 2 bis 25 Mol-%, insbesondere vorzugsweise 2 bis 10 Mol-%, bezogen auf Melamin, verwendet wird.

6. Melamin-Aldehyd-Kondensationsprodukt nach einem der vorstehenden Ansprüche, **dadurch gekennzeichnet, daß**, wenn ein Melaminderivat der Formeln (I) und (II) zusammen verwendet wird, das Verhältnis zwischen dem Melaminderivat der Formeln (I) und (II) in einem Bereich zwischen 9:1 und 1:3, vorzugsweise zwischen 4:1 und 1:1, am meisten bevorzugt in einem Bereich zwischen 4:1 und 2:1 liegt.

7. Melamin-Aldehyd-Kondensationsprodukt nach Anspruch 6, **dadurch gekennzeichnet, dass** die Melaminderivate der Formeln (I) und (II) in Form eines Produktgemisches verwendet werden, wie es nach ihrer Synthese, insbesondere der Synthese durch Transaminierung, erhalten wird.

8. Melamin-Aldehyd-Kondensationsprodukt nach einem der vorstehenden Ansprüche, **gekennzeichnet durch** einen Verformbarkeitsradius, wie er durch den Heißmanteltest bestimmt wird, zwischen 1 und 4 mm, vorzugsweise zwischen 1 und 3 mm, am meisten bevorzugt 2 und 3 mm.

9. Verfahren zum Erhalten eines Melamin-Aldehyd-Kondensationsprodukts nach einem der vorstehenden Ansprüche, umfassend die folgenden Schritte
- Bereitstellen einer Mischung aus mindestens einem Aldehyd und Melamin liefert,
- gleichzeitige oder spätere Zugabe von mindestens einem Melaminderivat der Formel (I) und/oder mindesten einem Melaminderivat der Formel (II),
- Einstellen des pH-Wertes der Mischung auf einen Bereich zwischen 7 und 11, vorzugsweise 8 und 10,
- Erhitzen des Reaktionsgemisches auf eine Temperatur zwischen 60°C und 120°C, vorzugsweise 70°C und 110°C, am besten 80°C und 100°C, bis eine klare Lösung erhalten wird,
- Rühren der Harzmischung bei erhöhter Temperatur, bis eine Wassertoleranz des Harzes bei 20°C von v(Wasser):v(Harz) zwischen 3:1 bis 0,3:1, vorzugsweise 2,5:1 bis 0,6:1, am meisten bevorzugt 2:1 bis 1:1, erreicht ist, und
- Abkühlen der Reaktionsmischung auf Raumtemperatur nach Abschluss der Reaktion und
- wahlweise Einstellung des pH-Wertes auf einen Bereich zwischen 8 und 10.

10. Verfahren nach Anspruch 9, **dadurch gekennzeichnet, dass** das Lösungsmittel aus einer Gruppe ausgewählt wird, die Wasser, einen Alkohol oder eine Mischung davon umfasst, wie eine Mischung, die 1-95 Gew.-% Wasser und 99-5 Gew.-% Alkohol, insbesondere Methanol, Ethanol, Isopropanol, umfasst.

11. Verfahren nach Anspruch 9 oder 10, **dadurch gekennzeichnet, dass** der Aldehyd aus einer Gruppe ausgewählt wird, die einen C1-C8-Aldehyd, insbesondere Formaldehyd, Acetaldehyd, Glyoxal, Dimethoxyethanal, umfasst.

12. Verfahren nach einem der Ansprüche 9 bis 11, **dadurch gekennzeichnet, dass** der pH-Wert unter Verwendung einer organischen oder anorganischen Base, vorzugsweise einer anorganischen Base, ausgewählt aus einer Gruppe, die NaOH, KOH, Na₂CO₃, K₂CO₃, NH₃ umfasst, eingestellt wird.

13. Verwendung eines Melamin-Aldehyd-Kondensationsprodukts nach den Ansprüchen 1 bis 8 als Imprägnierungsharz für Laminatpapiere und als Beschichtung wie Laminatbeschichtung oder für Dekorschichten von Postforming-Laminaten, Klebstoffe wie Klebstoffe für Holzwerkstoffplatten.

14. Holzwerkstoffplatten, insbesondere Spanplatten (PB), OSB, MDF, LDF oder HDF und Sperrholz, die ein Melamin-Aldehyd-Kondensationsprodukt nach einem der Ansprüche 1 bis 8 enthalten.

## Revendications

1. Produit de condensation de mélamine-aldéhyde pouvant être obtenu par
réaction d'au moins un aldéhyde, en particulier le formaldéhyde, avec
a) de la mélamine,
b) au moins un dérivé de mélamine de structure générale (I) 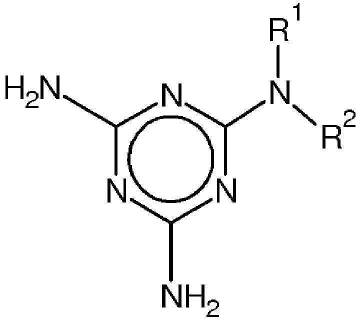 qui est utilisé en une quantité allant de 1 à 45% en moles par rapport à la mélamine, et
c) au moins un dérivé de mélamine de structure générale (II) 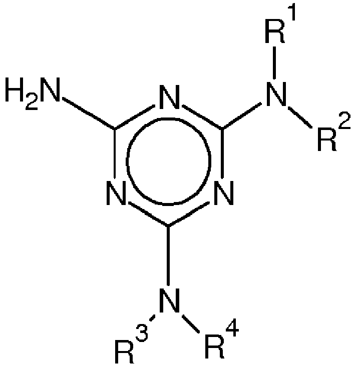
qui est utilisé en une quantité allant de 1 à 45% en moles par rapport à la mélamine,
dans laquelle R¹, R², R³ et R⁴ signifient indépendamment les uns des autres un alkyle en C₁ à C₂₀ linéaire ou ramifié, un cycloalkyle en C₅ à C₂₀, un aryle en C₅ à C₂₀, un aryle en C₅ à C₂₀ substitué par un alkyle en C₁ à C₂₀ ou des amines cycliques, ou dans laquelle R¹ et R² ou R³ et R⁴ forment ensemble un noyau alkyle, où dans chaque cas un ou plusieurs atome(s) de carbone peut/peuvent être substitué(s) par un ou plusieurs atome(s) d'oxygène, atome(s) de soufre et/ou atome(s) d'azote substitué(s) et/ou par un ou plusieurs groupe(s) du type -C(O)O-,-OC(O)-, -C(O)- et/ou -OC(O)O-, et/ou peut/peuvent être fonctionnalisé(s) par un ou plusieurs groupe(s) hydroxyle et/ou groupe(s) mercapto ; ou un amide d'un acide carboxylique ou un imide d'un acide dicarboxylique cyclique, et
dans laquelle R¹, R², R³ et R⁴ peuvent être identiques ou différents,
où le rapport molaire de l'aldéhyde et de la mélamine/les dérivés de mélamine est compris entre 1,8:1 et 1,3:1.

2. Produit de condensation de mélamine-aldéhyde selon la revendication 1, **caractérisé en ce que** R¹, R², R³ et R⁴ sont choisis dans un groupe comprenant un alkyle en C₁ à C₁₀ linéaire ou ramifié, un cycloalkyle en C₅ à C₁₀ ou un aryle en C₅ à C₁₀ substitué par un alkyle en C₁ à C₁₀ ou dans lequel R¹ et R² ou R³ et R⁴ forment dans chaque cas ensemble un noyau alkyle en C₅ à C₁₀, dans lequel dans chaque cas un ou plusieurs atome(s) de carbone peut/peuvent être substitué(s) par un ou plusieurs atome(s) d'oxygène, atome(s) de soufre et/ou atome(s) d'azote substitué(s) et/ou peut/peuvent être fonctionnalisé(s) par un ou plusieurs groupe(s) hydroxyle et/ou groupe(s) mercapto.

3. Produit de condensation de mélamine-aldéhyde selon la revendication 1 ou 2, **caractérisé en ce que** R¹, R², R³ et R⁴ sont choisis dans un groupe comprenant méthyle, éthyle, n-propyle, isopropyle, n-butyle, s-butyle, t-butyle, isobutyle, pentyle, cyclopentyle, hexyle, cyclohexyle, 2-éthyl-hexyle, octyle, décyle, stéaryle, phényle, toloyle, xyloyle, hydroxyéthyle, hydroxypropyle ou dans lequel R¹ et R² ou R³ et R⁴ font partie de la morpholine.

4. Produit de condensation de mélamine-aldéhyde selon l'une des revendications précédentes, **caractérisé en ce que** l'au moins un dérivé de mélamine de formule (I) est utilisé en une quantité allant de 2 à 25% en moles, en particulier de préférence de 2 à 10% en moles par rapport à la mélamine.

5. Produit de condensation de mélamine-aldéhyde selon l'une des revendications précédentes, **caractérisé en ce que** l'au moins un dérivé de mélamine de formule (II) est utilisé en une quantité allant de 2 à 25% en moles, en particulier de préférence de 2 à 10% en moles par rapport à la mélamine.

6. Produit de condensation de mélamine-aldéhyde selon l'une des revendications précédentes, **caractérisé en ce que** si dans le cas où des dérivés de mélamine de formules (I) et (II) sont utilisés ensemble alors le rapport entre les dérivés de mélamine de formules (I) et (II) se situe dans une plage comprise entre 9:1 et 1:3, de préférence entre 4:1 et 1:1, le plus préférablement dans une plage comprise entre 4:1 et 2:1.

7. Produit de condensation de mélamine-aldéhyde selon la revendication 6, **caractérisé en ce que** les dérivés de mélamine de formules (I) et (II) sont utilisés sous forme d'un mélange de produits tel qu'obtenu après leur synthèse, en particulier la synthèse par transamination.

8. Produit de condensation de mélamine-aldéhyde selon l'une des revendications précédentes, **caractérisé par** un rayon de formabilité tel que déterminé par l'essai au doigt incandescent compris entre 1 et 4 mm, de préférence entre 1 et 3 mm, le plus préférablement entre 2 et 3 mm.

9. Procédé d'obtention d'un produit de condensation de mélamine-aldéhyde selon l'une des revendications précédentes comprenant les étapes consistant
- à fournir un mélange d'au moins un aldéhyde et de la mélamine,
- à ajouter simultanément ou à un stade ultérieur au moins un dérivé de mélamine de formule (I) et/ou au moins un dérivé de mélamine de formule (II),
- à ajuster la valeur du pH du mélange dans une plage comprise entre 7 et 11, de préférence entre 8 et 10,
- à chauffer le mélange réactionnel à une température comprise entre 60°C et 120°C, de préférence entre 70°C et 110°C, le plus préférablement entre 80 °C et 100°C jusqu'à l'obtention d'une solution limpide,
- à agiter le mélange de résine à température élevée jusqu'à l'obtention d'une tolérance à l'eau de la résine à 20°C v(eau):v(résine) comprise entre 3:1 et 0,3:1, de préférence entre 2,5:1 et 0,6:1, le plus préférablement entre 2:1 et 1:1, et
- à refroidir le mélange réactionnel à température ambiante après achèvement de la réaction, et
- facultativement à ajuster la valeur du pH dans une plage comprise entre 8 et 10.

10. Procédé selon la revendication 9, **caractérisé en ce que**, le solvant est choisi dans un groupe comprenant l'eau, un alcool ou un mélange de ceux-ci, tel qu'un mélange comprenant 1 à 95% en poids d'eau et 99 à 5% en poids d'alcool, en particulier le méthanol, l'éthanol, l'isopropanol.

11. Procédé selon la revendication 9 ou 10, **caractérisé en ce que** l'aldéhyde est choisi dans un groupe comprenant un aldéhyde en C1 à C8, en particulier le formaldéhyde, l'acétaldéhyde, le glyoxal, le diméthoxyéthanal.

12. Procédé selon l'une des revendications 9 à 11, **caractérisé en ce que** la valeur du pH est ajustée en utilisant une base organique ou inorganique, de préférence une base inorganique choisie dans un groupe comprenant NaOH, KOH, Na₂CO₃, K₂CO₃, NH₃.

13. Utilisation d'un produit de condensation de mélamine-aldéhyde selon les revendications 1 à 8 comme résine d'imprégnation pour papiers stratifiés et comme revêtement tel qu'un revêtement stratifié ou pour des couches décoratives de stratifiés postformables, des adhésifs tels que des adhésifs pour panneaux à base de bois.

14. Panneaux à base de bois, notamment panneaux de particules (PB), OSB, MDF, LDF ou HDF et contreplaqué, comprenant un produit de condensation de mélamine-aldéhyde selon l'une des revendications 1 à 8.
